# EUROPEAN PATENT SPECIFICATION

(11) **EP 3 840 867 B1**
(45) Date of publication and mention of the grant of the patent: **05.01.2022**
(21) Application number: 19753132.0
(22) Date of filing: 20.08.2019
(51) Int. Cl.: B01F 3/04, B01F 13/08, B01F 15/00, B01F 15/02, A61K 9/12, B01F 13/00

(54) **METHODS, DEVICES, SYSTEMS AND KITS FOR PREPARING COMPOSITIONS FOR CARE AND REPAIR OF VARICOSE VEINS**
VERFAHREN, VORRICHTUNGEN, SYSTEME UND KITS ZUR HERSTELLUNG VON ZUSAMMENSETZUNGEN ZUR PFLEGE UND REPARATUR VON KRAMPFADERN
PROCÉDÉS, DISPOSITIFS, SYSTÈMES ET KITS POUR PRÉPARER DES COMPOSITIONS DE SOIN ET RÉPARATION DE VEINES VARIQUEUSES

(30) Priority: 21.08.2018 EP 18382621
(43) Date of publication of application: 30.06.2021
(73) Proprietor: Vascular Barcelona Devices, S.L., 08006 Barcelona (ES)
(72) Inventor: ROCHE REBOLLO, Enrique, 08034 Barcelona (ES); GALY, Jean-Baptiste, 08202 Sabadell, Barcelona (ES); LLUSÀ MELÉNDEZ, Guiu, 08018 Barcelona (ES); GREGO MAYOR, Federico, 08021 Barcelona (ES); GARCIA DE CASTRO, Arcadio, 28240 Hoyo de Manzanares (ES)
(74) Representative: de Rooij, Mathieu Julien
(86) International application number: PCT/EP2019/072242
(87) International publication number: WO 2020/038928

(56) References cited:
- WO-A1-2016/001847
- WO-A1-2017/096035
- US-A- 4 082 668
- US-A1- 2017 144 115

## Description

The present disclosure is related to the technical field of vascular medicine, and more particularly to the field of treatments for varicose veins and other vascular problems such as e.g. spider veins and / or haemorrhoids.

Specifically, the present disclosure relates to devices, systems, kits and methods for obtaining a foam, which can be used for the treatment of the affected veins. The present disclosure further relates to the composition of the foam itself obtained by any of the procedures described throughout the present disclosure, as well as the use of the devices, systems and kits in order to treat varicose veins and other vascular problems such as e.g. spider veins and/or haemorrhoids.

### BACKGROUND ART

Varicose veins occur when the venous valves (which prevent the backflow of blood) do not work properly. As a result, the vein walls are weakened, and they can become deformed and dilated. Due to the fact that the valves do not work properly, the blood may recirculate and short-circuits may be created. Subsequently, the veins may become progressively dilated. As a result, the varicose veins can become more visible, and can be full of bends and become more voluminous. The evolution of this pathology may lead to consequences beyond the mere cosmetic ones, such as discoloration of the skin, pain and swelling of the extremities due to the effect of the venous hypertension.

According to the Spanish Society of Angiology and Vascular Surgery (SEACV) in Spain, varicose veins affect from 30 to 33 % of the adult population in industrialized countries.

The veins most commonly affected are those located in the legs, although varicose veins may occur interiorly as well: e.g. varicose veins in the esophagus, around organs located in the pelvis (pelvic and ovarian varicose veins) or at or near the most distal part of the digestive tube, near the anus (haemorrhoids).

Nowadays, there are many different treatments and / or strategies in order to mitigate or eliminate these problems. Among them, we can find surgical methods. These surgical methods are related to the surgical extraction of the affected veins using classical surgery. This technique has been employed for over 100 years and is based on making different skin incisions with subsequent clamping, ligation and stripping of the venous segments affected. This technique is performed under spinal anesthesia (infiltration of the spinal space) in order to obtain the anesthesia of both limbs, as well as with local or nerve block anesthesia to anesthetize more limited areas.

In the late 1990's, there was an important advance regarding the treatment of this disease because the varicose veins were treated with less invasive techniques such as the endovenous techniques. Among these new methods, it is important to highlight intravenous procedures that apply heat through a catheter (endolaser or radio frequency systems).

These systems were able to reduce injuries due to the fact that the methods are usually performed under ultrasound guidance. The effect to the veins was produced by the release of heat or electricity from inside the vein, thus an internal injury into the vein may be produced. This way, a thrombosis of the veins was achieved.

Another technique is sclerotherapy by injection of a sclerosing foam. This technique (compared to the "Endolaser" or radiofrequency therapies) is even less aggressive, less painful, and needs no anesthesia (R Van den Bos et al. Endovenous therapies varicosites of lower extremity. A meta-analysis. J Vasc Surg 2009 Jan; 49 (1): 230- 9).

In summary, in the late 1990's and early 2000's a trend was found to minimize the aggressiveness of varicose veins treatments. Of all the new emerged techniques, sclerotherapy seems to be the less invasive and the one which can be applied to practically any type of varicose vein.

For this reason, in recent years, this procedure has been developed a lot further and there has been a growing interest from the scientific community in order to obtain methods suitable for the manufacture of a foam in a safe and convenient way.

Sclerotherapy involves the injection of a liquid with the ability to irritate the vascular endothelium (a thin layer or lining inside the vein that is in contact with the bloodstream).

This drug or liquid medicine can become a foam when shaken. The products internationally approved for this use are lauromacrogol (also known named as polidocanol and commercially available as etoxiesclerol^{®}), and sodium tetradecyl sulfate.

The advantage of using such a product as a foam is based on the enhancement of its effect due to the larger contact surface with the endothelial wall. The larger contact surface offers the possibility of dose reduction. Also the visibility of the drug as a foam using ultrasounds through the ultrasound scanner is improved (Schadeck M, Allaert FA. Duplex scanning in the mechanism of the sclerotherapy: Importance of the spasm. Phlebology 1995; Suppl1: 574-576).

The effect produced by the foam on the endothelium involves the injury of the cell layer of the vein, whereby thrombosis of its content is produced. Later, this vein suffers a fibrosis process (retraction and disposal) and the vein eventually may disappear after several months.

This process can be faster or slower depending on the size of the vein or the potency of the varicose agent. Therefore, it is sometimes necessary to apply several sessions on the vein.

Although there are several methods in order to eliminate or remove varicose veins, the less aggressive and less disabling treatment so far, and the one which can be used for a wide variety of pathologies is the ultrasound-guided Foam Sclerotherapy using polidocanol or other sclerosing agents. Sclerotherapy is the least invasive treatment of varicose veins known today as it can be performed in a physician's office and in a completely ambulatory way. Therefore, the present disclosure is focused on the use of this technique.

In 1995, Dr. Juan Cabrera presented the results of the application of a foam that he had developed with his son, the pharmacist Juan Cabrera (Cabrera J. et al. "Treatment of Varicose Long Saphenous Veins of Sclerosants in Microfoam Form With: Long - term Outcomes". Phlebology (2000) 15; 19-23). This foam was characterized by its density and high solubility thanks to the use of a mixture of physiological gases.

Furthermore, he managed to achieve a type of foam with very small and uniform bubble size, thanks to his method of using a mixer. By using a mixture of physiological gases, the foam had greater security and stability. This foam was called "*microfoam*" due to the small bubble size, uniformity and stability.

Shortly after, the maximum popularization of the sclerosing foam came from Lorenzo Tessari (Tessari L., Cavezzi A., Frullini A., Preliminary experience with a new sclerosing foam in the treatment of varicose veins. Dermatol Surg 2001 Jan; 27 (1): 58-60) who published his experience using a foam easily manufactured through a procedure called "*The Tessari Method*". The method consists in the agitation of a sclerosing liquid using two syringes connected via a three-way tap. By means of successive alternately movements with each of the syringes connected to the gas/liquid mixture, a mix of foam, located at the inner part of the syringe, was achieved. However, this manufacturing technique, in spite of being the most widespread is not the most effective, since a relatively unstable and heterogeneous foam is obtained.

In recent years numerous articles and papers have been published about safety profiles, side effects and potential complications arising from the use of these products. Thus, it appears demonstrated that the best foam used is the one whose gas is a mixture of O₂/CO₂ at different concentrations. With this arrangement, the solubility and diffusion in blood is very high as opposed to atmospheric gas foams. Additionally, the foam stability is linked to the size of the bubble. Also, it has been found that the foam is more stable when the bubble diameter is more homogeneous.

As has already been mentioned, although there are a lot of manufacturing systems of sclerosing foam, the most common foam (and the one which is normally used around the world) is the foam obtained with the Tessari method. However, this method has many problems of standardization and homogenization. This system consists of mixing air flow with the selected liquid, either polidocanol or sodium tetradecyl sulfate (commercially known as sotradecol ^{®}). This foam can have medium size bubbles, but of irregular size and the foam becomes unstable after a few seconds of its formation. Additionally, the use of atmospheric gas as a vehicle for the sclerosing foam limits the foam administrable per session.

US2017144115 discloses a container for the production of a foamed sclerosant composition, kits and systems including such a container, and methods for preparing a foamed sclerosant composition using such containers. In an aspect, the container comprises a container body and a mixing element disposed in the container body, such that a foaming space is defined in an interior of the container body between the sidewalls and the mixing element. The mixing element may be configured to be operatively coupled with a rotating actuator without the actuator reaching the foaming space. In particular, the mixing element may have a magnetic core, such that the mixing element can be set in motion when placed on a magnetic stirrer.

WO2017085209 relates to a container for the production of a foamed sclerosant composition, to kits and systems including such a container, to methods for preparing a foamed sclerosant composition using such containers, and to foamed sclerosant compositions obtainable by such methods. In an aspect, the container comprises a sealed sterile container body having one or more sidewalls extending between a top and a bottom of the container body and defining a foaming space. The container further comprises a mixing element disposed in the container body. The container contains a previously introduced gas and liquid sclerosant composition. The mixing element is configured to be operatively coupled with a rotatable actuator without the actuator reaching the foaming space. A medical professional may select the appropriate quantity and concentration for every treatment.

These documents disclose methods and devices that facilitate quick production of good quality sclerosant foam with relatively simple tools and in a highly sterile manner.

However, there is still a need for systems and methods that further improve the production of sclerosant foam.

### SUMMARY

In a first aspect, the present disclosure provides a container for the production of a foamed sclerosant composition according to claim 1. the container comprising a sealed sterile container body for foaming comprising one or more sidewalls extending between a top and a bottom of the container body, a foaming space being defined in an interior of the container body. The foaming space contains a substantially sterile gas and a mixing element for rotating on the a bottom surface of the interior of the container body and comprising a magnetic part and a shape suitable for mixing, and configured to be operatively coupled with a magnetic stirrer without the magnetic stirrer entering into the foaming space,. The container comprises a female coupling member for mating with a tip of a syringe, the female coupling member being located in one of the sidewalls of the container body, at or near the bottom surface of the interior of the container body such that the syringe can be used for extracting the foamed sclerosant composition. The container further comprises a pressure equalizer for equalizing a pressure inside the foaming space and outside the foaming space.

In accordance with this aspect, a container is provided which is specifically suited for the substantially sterile preparation of a sclerosant foam in which a syringe may be used for the introduction of a liquid sclerosant composition. The introduction of a liquid sclerosant composition can produce an overpressure inside the foaming space, i.e. the (atmospheric) pressure inside the foaming space is higher than the ambient pressure.

A syringe may be used for aspiration or extraction of foam from the foaming space as well. The same syringe may then be used for injection into a patient's vein. The overpressure on the inside of the foaming space may provoke problems during aspiration, in that air or gas bubbles are present in the syringe. In order to avoid such problems, a pressure equalizer is provided which can decrease or avoid the overpressure in the foaming space.

In another aspect, the present disclosure provides a kit according to claim 10 for the preparation of a foamed sclerosant composition comprising such a container and further comprising a syringe, specifically a syringe pre-filled with a liquid sclerosant composition.

In examples, the syringe may be a Luer lock syringe comprising one of the following volumes: 2.5 cm³, 5 cm³, 10 cm³, or 20 cm³. In examples, the syringe may comoprise 2 - 4 ml of a liquid sclerosant composition. In some examples, the volume ratio gas : liquid in the foaming space once the liquid sclerosant composition is introduced into the container body is between 7.5 : 1 and 20 : 1.

The liquid sclerosant composition is a solution of a drug in distilled water or a saline.

Also described, but not claimed is a system which comprises such a container and an actuator configured to be operatively coupled to the mixing element. The actuator may in particular be a magnetic stirrer.

In a further aspect, a method for preparing and extracting a foamed sclerosant composition according to claim 11 is provided. The method comprises providing a container having a sealed sterile container body for foaming comprising one or more sidewalls extending between a top and a bottom of the container body, a foaming space being defined in an interior of the container body, the foaming space containing a sterile gas and a mixing element configured to be operatively coupled with a rotatable actuator without the actuator entering into the foaming space, wherein the container furthermore comprises a female coupling member for mating with a syringe, and a pressure equalizer for equalizing a pressure inside the foaming space and outside the foaming space. The method further comprises providing a syringe filled with a liquid sclerosant composition, mating the syringe with the female coupling member and injecting the liquid sclerosant composition into the foaming space, operating the actuator to rotate the mixing element and create a foamed sclerosant composition, and extracting the obtained foamed sclerosant composition.

In a method according to this aspect, a substantially sterile and relatively easy method for preparing and extracting a foamed sclerosant composition is provided allowing the coupling of a syringe with the container having the foaming space.

In examples, the syringe may remain mated with the coupling while the foam is being created. In examples, the obtained foamed sclerosant composition may be extracted by aspirating the syringe.

In examples, the actuator may be operated to rotate the mixing element at a first speed of rotation of 5,000 RPM or below, and preferably rotating the actuator to rotate the mixing element at a first speed of rotation of between 2,000 and 4,500 RPM during a first period of time. In some of these examples, the method may comprise rotating the actuator to rotate the mixing element for a first period of time at a first speed of rotation with a varying speed. Herein, the first period of time may be between 15 and 90 seconds, specifically between 30 and 75 seconds, more specifically between 30 and 60 seconds.

In examples of the methods, the actuator may be operating while the obtained foamed sclerosant composition is being extracted. In examples, the actuator may be operated at a first speed of rotation to create a foamed sclerosant composition, and the actuator may be operated at a second speed of rotation while the foamed sclerosant composition is being extracted, wherein the first speed of rotation is different from the second speed of rotation.

The second speed of rotation may be lower than the first speed of rotation. In some of these examples, the method may comprise rotating the actuator to rotate the mixing element at a second speed of rotation of 1,500 RPM or below, specifically between 500 - 1,200 RPM, more specifically around 1,000 RPM. The method may further comprising releasing an overpressure inside the foaming space before extracting the obtained foamed sclerosant composition.

In examples of these methods, the liquid sclerosant composition may be a solution of the sclerosant drug in water, optionally distilled water, or a saline. In examples, a concentration of the drug is 0.20 - 3.0 % (w/v). Specifically, the concentration of the drug may be 0.20 - 0.50 % (w/v).

In yet a further non-claimed aspect, a foamed sclerosant composition obtainable by such a method is provided.

In some examples, the female coupling member or "socket" of the container body comprises a Luer taper. A female coupling member as used throughout the present disclosure may be regarded as a coupling member with a suitable receptacle or opening for receiving a male coupling member, and in the present case the male coupling member of a distal end of a syringe. The female coupling member may have the shape of a socket or a sleeve.

The Luer taper is a standardized system of small-scale fluid fittings used for making leak-free connections between a male-taper fitting and its mating female part on medical and laboratory instruments, including e.g. syringe tips.

The Luer taper of the female coupling member may be a female Luer slip. Alternatively, a Luer-lock may be used. Luer lock fittings are securely joined by means of a tabbed hub on the female fitting which screws into threads in a sleeve on the male fitting. Slip tip (Luer-slip) fittings simply conform to Luer taper dimensions and are pressed together and held by friction (they have no threads).

In some examples, the female coupling member for mating with a syringe further may comprise a one-way valve for the introduction of a liquid sclerosant composition. The one-way valve may ensure that the introduction of a liquid sclerosant composition can be done without compromising the sterile environment in the foaming space. The one-way valve may be a simple sheet or foil which is cantilever mounted to allow pivoting and thereby provide a one-way access to the foaming space. In some examples, the one-way valve may be arranged downstream from the point of injection of the liquid composition.

In some examples, the pressure equalizer may be a pressure release. The pressure release may be configured to release pressure only after a sclerosant foam has been created.

In some examples, the pressure equalizer or release may be manually activated. In some of these examples, the pressure equalizer or release comprises an orifice in the container body and a cover. The cover may be removable, releasable, or breakable. A user may simply remove the cover in order to reduce or avoid the differences in pressure in the inside of the foaming space, and a user can do so at a suitable moment. In some examples, instead of removing the cover, the cover may be broken. In other examples, the pressure equalizer or release may be an (automatic) release valve. "Automatic" herein means is that no manual activation by a user or healthcare professional is necessary.

In some examples, the mixing element configured to be operatively coupled with a rotatable actuator may be a disc comprising a magnetic part and a shape suitable for mixing. Such a mixing element can be activated and controlled by providing a changing magnetic field. In particular, the rotatable actuator may be configured to cause a rotating magnetic field, and optionally may be a magnetic stirrer.

In some examples, the gas may be a physiological gas, and optionally is a mixture of O₂ and CO₂. A percentage of O₂ may be between 70%-50%, the remainder being CO₂. In other examples, the gas may be air. The foam prepared with physiological gases may be suitable for the treatment of certain patients and/or veins which cannot be treated by foam prepared with (sterile) air.

In some examples, the syringe used for the introduction of the liquid sclerosant composition remains mated with the female coupling member or "socket" while the foam is being created. If the syringe remains coupled with the container, the chances of contaminating the foam are reduced. After the foam has been created, the same syringe may be used for aspiration or extraction of the foam. Depending on how much foam is extracted, a lower pressure may be created inside the foaming space than outside the foaming space. The pressure equalizer may also be useful to again equalize the pressures so that a good quality foam may be extracted.

In some examples, the actuator may be operated at a first speed of rotation to create a foamed sclerosant composition, and the actuator is operated at a second speed of rotation while the foamed sclerosant composition is being extracted, wherein the first speed of rotation is different from the second speed of rotation. In particular, a first high speed of rotation may be used for rapid preparation of foam of good quality (e.g. small bubbles), and the rotation continues as the foam is being extracted. The continued rotation forces the created foam to the outer area of the foaming space, from where it can be aspirated.

In some examples, the foamed sclerosant composition obtainable by any of the methods described herein may be for use in the treatment of varicose veins, spider veins or haemorrhoids. The ratio of liquid : gas in the sclerosant foam may be between 1:4 and 1:7.

Also disclosed but not claimed is a magnetic stirrer having one or more magnets for creating a rotating magnetic field, wherein the magnetic stirrer has a control, the control including a processor and memory, wherein the memory includes instructions corresponding to one or more predetermined rotation programs, such that when the instructions are executed by the processor, the magnets create a rotating magnetic field according to the predetermined rotation programs, the predetermined rotation programs corresponding to rotations defined in any of the examples disclosed herein.

The one or more magnets may be rotating magnets, or static electromagnetc. The magnetic stirrer may further comprise a user interface, wherein the user interface enables selection by a user of the predetermined rotation programs. The user interface may include a touch screen display. The predetermined rotation programs may be identified by one or more of the following: the gas in the foaming space, and the liquid sclerosant composition, specifically a concentration of the drug in the liquid sclerosant composition.

### BRIEF DESCRIPTION OF THE DRAWINGS

Particular implementations of the present disclosure will be described in the following by way of non-limiting examples, with reference to the appended drawings, in which:
Figures 1A - 1E schematically illustrate an example of a container for producing a foamed sclerosant composition;
Figures 2A - 2F schematically illustrate an example of a mixing element which may be used in containers for preparing a foamed sclerosant composition; and
Figure 3 schematically illustrates a sequence of steps in an example of a method for the production of a foamed sclerosant composition.

### DETAILED DESCRIPTION

The expression "therapeutically effective amount" as used herein, refers to the amount of the foamed composition that, when administered, is sufficient to treat the diseases to which it is addressed. The specific dose which depends on both the volume and the drug concentration of the foamed sclerosant composition to obtain a therapeutic benefit may vary depending on the particular circumstances of each patient.

As previously mentioned, a non-claimed aspect of the present disclosure relates to a foamed sclerosant composition obtainable by any of the methods herein described. The expression "obtainable by" is used herein for defining the foamed sclerosant composition by its preparation process. In particular, it refers to the foamed composition that can be obtained through the preparation process which comprises the previously commented steps. For the purposes of the present disclosure, the expressions "obtainable", "obtained" and similar equivalent expressions are used interchangeably and, in any case, the expression "obtainable" encompasses the expression "obtained". Throughout the present disclosure, the terms sclerosant and sclerosing are used interchangeably. Similarly, sclerosing foam, and foamed sclerosant composition are used interchangeably as well.

For the purposes of the present disclosure, the term foamed sclerosing composition refers to a composition of a foam capable of bringing about a sclerosing effect, i.e. a composition for use as a medicament for intravenous injection, which is capable of causing an injury to the vessel wall by endothelial vacuolation of the epithelial cell membrane (the layer in contact with the bloodstream). Thus, the foamed sclerosing composition irritates the inner surface of the vein just producing the formed thrombus formation by platelets and aggregates. Similarly, the term liquid sclerosing composition refers to a composition in liquid form including a sclerosing agent. The liquid sclerosing composition forms an ingredient to obtain the foamed sclerosing composition. The sclerosing compositions according to examples of the present disclosure comprise a sclerosing agent and also a suitable vehicle which can be injected without toxicity in the affected veins. In some examples, the liquid is selected from sterile water (particularly distilled water) and physiological saline. Examples of sclerosing agents that can be present in the sclerosing compositions of examples of the present disclosure include, without limitation, polidocanol, sodium tetradecyl sulfate, chromated glycerin, hypertonic saline, sodium morrhuate and sclerodex (hypertonic saline in combination with dextrose). In a particular example, the sclerosing composition used comprises polidocanol and water. In another embodiment, the sclerosing composition further comprises glycerin.

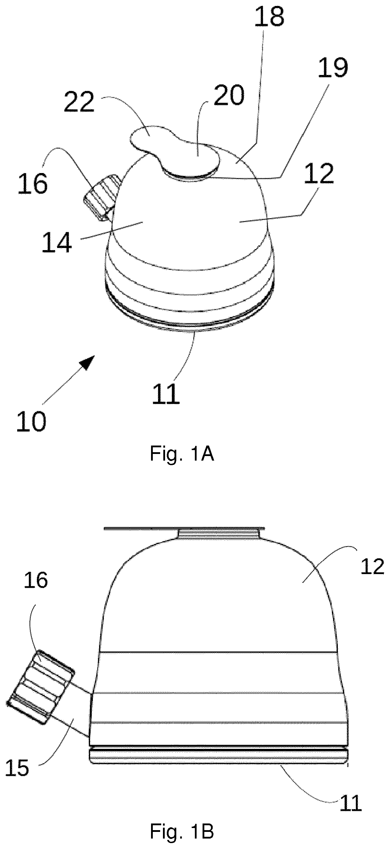

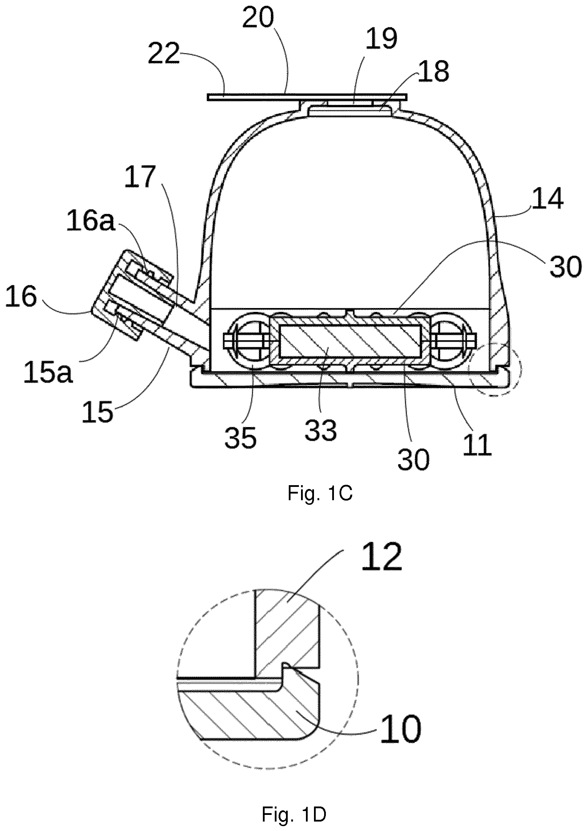

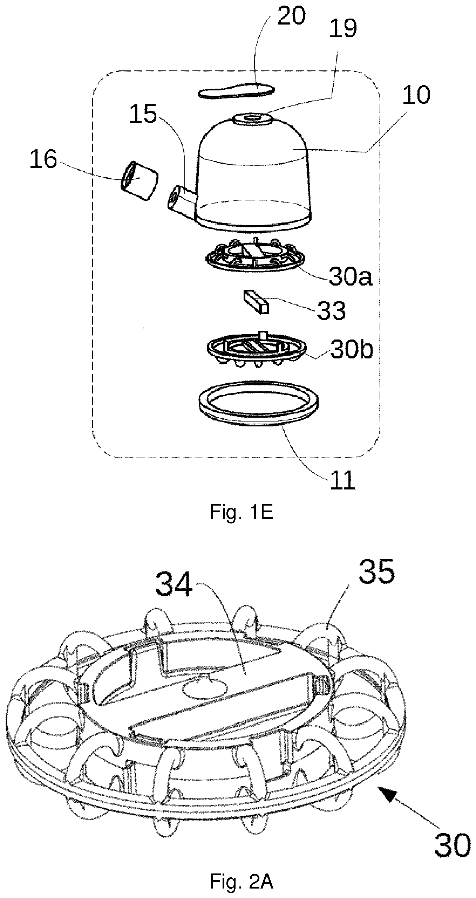

Figures 1A - 1E schematically illustrate an example of a container for producing a foamed sclerosant composition. A container 10 according to this example may include a dome shaped container body 12 with a separately manufactured bottom 11. The dome shaped container body 12 may have a top 18 with an orifice 19. In this example, the orifice is closed by a removable cover 20.

The dome shape is formed by a curved side wall 14. In the interior of the container 10 a foaming space is defined. The container 10, both dome shaped body 12 and bottom 11, may be made from a suitable polymer such as Polyethylene terephthalate (PET) or other suitable materials such as plastics generally accepted in manufacture of medical devices.

In some examples, the material of which the container is made is transparent to allow visual inspection of the formation of the foam. The dome shaped container body 12 and the bottom part 11 may be made by injection molding within the same mold or by separate molds. Other manufacturing processes could also be used.

If manufactured separately, the bottom 11 and the dome shaped body 12 may be joined by ultrasonic welding or other appropriate ways for joining plastic components.

The container 10 according to this example may be sterilized and wrapped. For example, plastic foil or wrapper may be provided around the container as delivered to a medical professional.

A sterile gas may be introduced into the foaming space during manufacture and assembly. The gas may be air but may also be a mixture of physiological gases, e.g. a mixture of carbon dioxide (CO₂) and oxygen (O₂). As provided, the foaming space also includes a mixing element 30 (see figure 1c). The sterile container with previously introduced gas and mixing element may thus be provided to a medical professional. In the shown example, the device of the present disclosure is sealed and closed, accesses to the interior of the container provided only by access ports, sockets and operational orifices that are located for best performance and reproducible production of improved foam. Restricted access to the interior of the device reduces risks of misuse and incorrect operation.

As shown in figure 1b, the container 10 according to this example further includes a female coupling member in the form of a socket 15 with a stopper or lid 16. The socket 15 may comprise external threads 15a and the stopper 16 may comprise mating threads 16a for easy attachment and detachment of the stopper. Alternative joints for socket and stopper may however also be foreseen, e.g a press or snap fit.

The coupling socket 15 in this example comprises a Luer taper, and particularly a Luer slip. As shown in figure 1c, the Luer taper 17 may be sized such that it can mate with a tip of a syringe, in particular a hypodermic syringe. The tip of the syringe may be pressed into the Luer taper to provide a leak free fitting. In some examples, the syringe may be rotated to lock the syringe in the socket by means of a Luer lock.

In this example, the socket 15 is integrally formed with container 10 and in particular is provided on sidewall 14, in proximity to bottom 11. In some examples, the center of socket 15 is on the sidewall 14 up to 20 mm away from the bottom 11 of the container 10. Specifically, the center of socket 15 may be on the sidewall 14 up to 10 mm away from the bottom 11 of the container 10. However, other suitable positions for coupling socket 15 may be envisaged. The stopper 16 may be made from polyethylene or other suitable materials.

Although not shown in this specific example, the socket 15 or its connection to the foaming space may include a one-way valve, or any other element that facilitates passage from a liquid inside the syringe, and substantially impedes passage of gas from the inside to the outside.

In this example, the coupling socket 15 has an inclination with respect to a horizontal plane for easy insertion and extraction from the syringe.

Filling of the foaming space with a suitable sterile gas may take place through the same socket 15 that is used for injection of a sclerosant liquid composition, and potentially the same socket that is used for extracting the foam. An example of an assembly process is the following: after injection molding of the container 10, and before attachment of the bottom 11, the mixing element 30 is introduced into the foaming space of the container, and the housing is then closed. Sterile gas may be introduced through a suitable valve coupling which allows the introduction of the sterile gas (e.g. air, and/or a mixture of physiological gases) and evacuation of the gas previously present.

The container 10 includes a pressure equalizer, and in this specific example, a pressure release. In this specific example, an orifice 19 with a releasable cover 20 is provided as the pressure release. The releasable cover may be heat sealed over the orifice. In other examples, the pressure release may be a relief valve, or an orifice with a breakable cover or tab. The function of the pressure release is to balance a pressure inside container 10 and a pressure outside the container. Equilibration of pressures can avoid potential formation of undesirable large bubbles in the sclerosant foam.

Orifice 19 may have a relatively small diameter, sufficient for gas exchange. Without limitation, orifice 19 can be 0.5 to 5 mm in diameter. Specifically, orifice 19 may be between 1 and 3 mm in diameter.

Orifice 19 may particularly be located where it is not exposed to the foam created, for example at a high point of container 10. In this example, orifice 19 is covered by a releasable cover 20 such as an aluminum foil sealed over orifice 19. As may be seen for example in figures 1B and 1C, the cover 20 may have a shape adapted for easily pulling off the cover and may include a handle or grip 22 which a user may grip for handling. The grip may be provided at one end of the cover 20, whereas the opposite end may seal the orifice 19. Orifice 19 may also include a filter element or microbial barrier to reduce potential exposure of the foam to airborne particles and microbes upon equilibration of pressures and withdrawal of the foam.

Opening of orifice 19 may be carried out at different moments in the operation of the device. In a specific example disclosed herein orifice 19 is maintained closed in operation during initial formation of the foam and is only exposed shortly before withdrawal of the foam contained in container 10. Orifice 19, or in general the pressure equalizer or release, may be configured such that it only becomes operational immediately prior or simultaneously to the withdrawal of the foam.

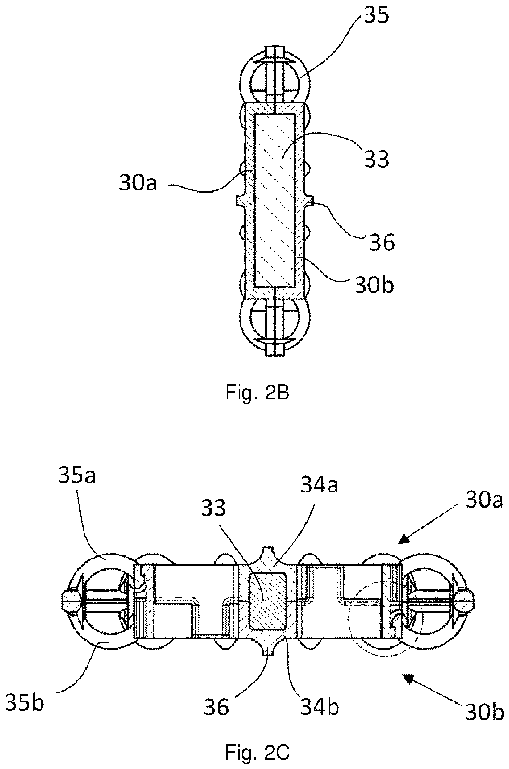

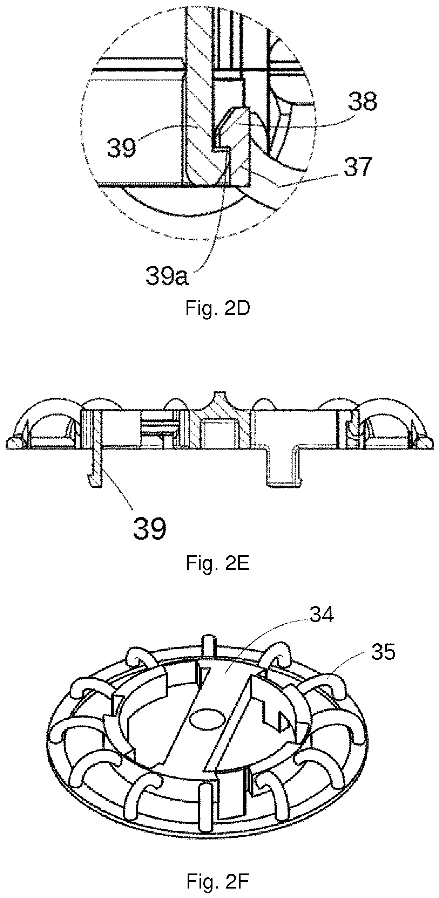

Details of mixing element 30 may particularly be seen in figures 2A - 2F. The mixing element 30 in this example may be a disc and, as may be seen e.g. in figure 2b, it may comprise a magnetic element 33, in particular a magnetic core. The disc may comprise an upper disc and a lower disc, configured to be coupled to each other, the upper and lower disc defining a compartment for housing the magnetic part. The upper and lower disc may be configured to be snap fitted together.

As shown e.g. in figure 2a, the magnetic element may be provided in a receptacle 34, e.g. a centrally arranged receptacle 34. The magnetic element 33 may be a permanent magnet or may be made from a (soft) ferromagnetic material. When using a (soft) ferromagnetic material, rather than a permanent magnet, the element 33 obtains its magnetic properties because of its arrangement in the presence of a magnet, e.g. the magnet of a magnetic stirrer. The magnetic properties then disappear when the magnet is removed. The magnetic element 33 may be made e.g. from neodymium, or other materials with good magnetic properties. In another example, the magnetic element may be made of stainless steel.

Around an outer perimeter of the disc a plurality of suitably shaped features for mixing may be provided. Such features may include e.g. blades, rings or vertical or horizontal teeth, which ensure mixing a liquid and gas by creating sufficient turbulence and mixing of the various components as the disc is rotated. Mixing may be increased by forced passage through openings or spaces in the disc.

The disc may be freely arranged, i.e. not supported or suspended by any element on the sidewall or the top of the container. The mixing element 30 may comprise a central protrusion 36 which is configured to reduce contact and friction between a bottom of the mixing element with the bottom of the container. The protrusion 36 may be tapered and pointed. When the mixing element starts to rotate, the protrusion 36 maintains contact with the bottom of the container body.

In this particular example, ring shaped mixing features 35 are shown, but it should be clear that many other shapes may be suitable.

In this particular example, as shown e.g. in figure 2c, the disc may be made by assembly of an upper half 30A and lower half 30B. Each of the halves 30A, 30B may include half rings 35A, 35B, half compartments 34A, 34B and suitable coupling features. Half compartment 34A, once coupled with half compartment 34B forms a receptacle for the magnetic element 33.

Each of the halves may be made from a suitable polymer, such as polyethylene and may be made e.g. by injection molding. As may be seen e.g. in figure 2d, the halves may include suitable joining features, e.g. fingers 37 with bulging tips 38 which can snap fit over corresponding bulges 39A on a sidewall 39 of the other half.

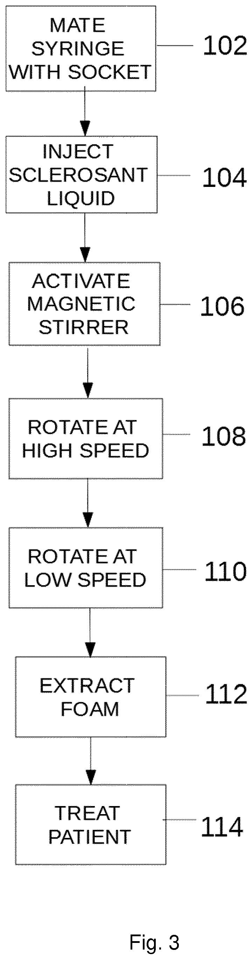

One example of preparing of a sclerosant foam with a container 10 and mixing element 30 may be illustrated with reference to figure 3.

A container according to the previously shown example or similar may be provided. A syringe filled with a sclerosant liquid composition may be provided as well. In an example, such a syringe may be provided as a pre-filled syringe, possibly as part of a kit also comprising container 10. Alternatively, the syringe may be filled with the sclerosant liquid immediately prior to the preparation of the foam.

In one particular example, the sclerosing composition may comprise a solution of a sclerosing agent, such as e.g. polidocanol, in a liquid, such as water or physiological saline, at a concentration from 2 mg to 30 mg in 1 mL liquid (which corresponds to 0.20 - 3.0 %) (w/v). In another example, the sclerosing composition may comprise a solution of a sclerosing agent, such as polidocanol, in a liquid, such as water or physiological saline, at a concentration from 2 mg to 5 mg in 1 mL liquid (which corresponds to 0.20 - 0.50 % (w/v)). With the devices and methods described herein, it has been found that even at very low concentrations e.g. 0.2% or 0.3% (w/v), still a stable foam may be obtained, contrary to e.g. Tessari's method.

In another particular example, the sclerosing composition may comprise a solution of polidocanol in water or physiological saline at a concentration of 5 mg/mL.

In another particular example, the sclerosing composition may comprise a solution of polidocanol in water or physiological saline at a concentration of 20 mg/mL.

In another particular example, the liquid composition may include Sodium tetradecyl sulfate (STS) at a concentration of 0.2 - 3%.

The volume of the syringe may be e.g. 5 cc, 10 cc or 20 cc. The syringe may comprise e.g. 2 ml or more of liquid sclerosant composition.

At block 102, the syringe may be mated with socket 15 by insertion of the syringe. Prior to this, the stopper 16 is removed from the socket 15. Immediately after removing stopper 16, the syringe may be introduced to avoid any contamination. In further examples, the coupling socket or container body may include a one-way valve or a selectively displaceable element to avoid any contamination. In yet further examples, the container may be provided with a slight overpressure (i.e. higher than ambient pressure) to avoid potential contamination once stopper 16 is removed. Combinations thereof are also possible. In examples, the stopper 16 may be configured for single use only. I.e. the stopper 16 may be in the form of a lid or seal that is broken, damaged or otherwise made unusable when removed.

Once a leak free fitting between the syringe and the socket 15 is established, the liquid composition inside the syringe may be injected at block 104 and can enter the foaming space. Because liquid is introduced into a closed system a slight overpressure can be created inside the foaming space, that is, the pressure inside the foaming space is higher than the external atmospheric pressure. Any air or gas inside the syringe may also be injected into the foaming space, although this may be done at any moment prior to extraction of the foam.

The previously described actions may be carried out in particular after placement of container 10 on a magnetic stirrer. This may be a standard magnetic stirrer which may typically be found in many laboratories. In some other examples, the magnetic stirrer may be specifically adapted to the container and include a receptacle for receiving the container. A slight press fit between the container and the receptacle may be provided.

Then, at block 106, the magnetic stirrer may be activated. The mixing element may thus be set in rotation. The mixing element, e.g. the disc with magnetic element that was previously described may rotate on the bottom of the container. Depending on the type of foam to be created, depending on the liquid sclerosant composition, and depending on the gas in the foaming space, different revolution speeds and times may be used. In some examples, the magnetic stirrer may include a memory with a number of predefined programs suitable for different foams, such that a user only needs to select a suitable program.

It is noted that it can be advantageous not to remove the syringe during the formation of the foam and maintain it coupled to the container.

In particular examples, the magnetic stirrer is first set to a high first speed at block 108, which is reached in a short time, for example a few seconds. Such a high first speed may be between 2,000 and 5,000 RPM, and particularly between 2,500 and 4,000 RPM. After as little as 15 seconds, foam may be created.

In order to increase the stability and quality of the foam, the high speed of rotation may be maintained however. After a first time period, which may be 30 - 90 seconds, particularly around 60 seconds, the speed of the magnetic stirrer may be reduced to a second speed at block 110.

The second speed may be chosen such that the foam that has been created is pushed to the outer perimeter of the foaming space due to centrifugal forces. The second speed may be lower than 1,500 RPM. In an example, the second speed may be between 200 and 1,200 RPM, specifically around 1,000 RPM.

Before extraction of foam at block 112, the releasable cover 20 may be removed from the pressure release orifice 19 to equilibrate the overpressure inside with the outside atmospheric pressure. Preferably, orifice 19 is maintained open during extraction of the sclerosant foam from the container to avoid creation of an underpressure inside the foaming space and syringe during the extraction. Equilibration of pressures is directed towards avoiding potential formation of undesirable large bubbles in the sclerosant foam within the collection syringe.

After extraction of the sclerosant foam, the foam may be used in the treatment of a patient at block 114.

The volume of container 10 as disclosed herein may be, for example, 30 ml. Such a container was used in various experiments. With methods according to the examples disclosed herein, approximately 10 ml of stable foam was obtained and easily extracted when introducing 2 ml of liquid sclerosant composition (of any of the hereinbefore mentioned concentrations).

When introducing 4 ml of liquid sclerosant composition, about 20 ml of stable foam was obtained and easily extracted.

A container according to any of the disclosed examples may be enclosed in a sterile packaging.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A container (10) for the production of a foamed sclerosant composition, the container comprising:
a sealed sterile container body (12) for foaming comprising one or more sidewalls (14) extending between a top (18) and a bottom (11) of the container body (12), a foaming space being defined in an interior of the container body,
the foaming space containing a sterile gas and a disc (30) configured for rotating on the bottom of the container body and comprising a magnetic part (33) and a shape suitable for mixing, and configured to be operatively coupled with a magnetic stirrer without the magnetic stirrer entering into the foaming space, **characterized in that**
the container comprises a female coupling member (15) having a Luer taper for mating with a tip of a syringe, the female coupling member being located in one of the sidewalls (14) of the container body, at or near the bottom surface of the container body such that the syringe can be used for extracting the foamed sclerosant composition and
a pressure equalizer (19, 20) for equalizing a pressure inside the foaming space and outside the foaming space.

2. A container (10) according to claim 1, wherein the female coupling member (15) comprises a lid (16) for closing the coupling.

3. A container according to any of claims 1 - 2, wherein the female coupling member (15) for mating with a syringe further comprises a one-way valve for the introduction of a liquid sclerosant composition.

4. A container according to any of claims 1 - 3, wherein the pressure equalizer (19, 20) is a pressure release.

5. A container according to claim 4, wherein the pressure release comprises an orifice (19) in the container body and a releasable cover (20).

6. A container according to claim 5, wherein the orifice (19) is located at the top (18) of the container body.

7. A container according to claim 5 or 6, wherein the orifice (19) has a diameter of 1 mm - 3 mm.

8. A container (10) according to any of claims 5 - 7, wherein the releasable cover (20) comprises a grip (22) for pulling off the releasable cover.

9. A container (10) according to any of claims 1 - 8, wherein the disc comprises an upper disc and a lower disc, configured to be coupled to each other, the upper and lower disc defining a compartment for housing the magnetic part.

10. A kit for the preparation of a foamed sclerosant composition comprising a container (10) according to any of claims 1 - 9, further comprising a syringe, specifically a syringe pre-filled with a liquid sclerosant composition.

11. A method for preparing and extracting a foamed sclerosant composition comprising:
providing a container (10) having:
a sealed sterile container body (12) for foaming comprising one or more sidewalls (14) extending between a top (18) and a bottom (11) of the container body (12), a foaming space being defined in an interior of the container body (12), the foaming space containing a sterile gas and a mixing element (30) configured to be operatively coupled with a rotatable actuator without the actuator entering into the foaming space, wherein
the container furthermore comprises a female coupling member (15) for mating with a syringe, **characterized in that**
the container comprises
a pressure equalizer (19, 20) for equalizing a pressure inside the foaming space and outside the foaming space; and the method further comprises:
providing a syringe filled with a liquid sclerosant composition;
mating (102) the syringe with the female coupling member (15) and injecting (104) the liquid sclerosant composition into the foaming space;
operating (106, 108, 110) the actuator to rotate the mixing element (30) and create a foamed sclerosant composition,
extracting (112) the obtained foamed sclerosant composition.

12. A method according to claim 11, wherein the syringe remains mated with the female coupling member (15) while the foam is being created, and optionally wherein the obtained foamed sclerosant composition is extracted by aspirating the syringe.

13. A method according to claim 11 or 12, wherein the actuator is operated (108) to rotate the mixing element at a first speed of rotation of 5,000 RPM or below, and preferably rotating the actuator to rotate the mixing element at a first speed of rotation of between 2,000 and 4,500 RPM during a first period of time.

14. A method according to claim 13, wherein the first period of time is between 15 and 90 seconds, specifically between 30 and 75 seconds, more specifically between 30 and 60 seconds.

15. A method according to any of claims 11 - 14, wherein the actuator is operating (110) while the obtained foamed sclerosant composition is being extracted at a second speed, wherein the second speed of rotation is lower than the first speed of rotation.

## Patentansprüche

1. Behälter (10) für die Herstellung einer geschäumten sklerosierenden Zusammensetzung, wobei der Behälter umfasst:
einen versiegelten sterilen Behälterkörper (12) zum Aufschäumen, der eine oder mehrere Seitenwände (14) aufweist, die sich zwischen einer Oberseite (18) und einer Unterseite (11) des Behälterkörpers (12) erstrecken, wobei ein Aufschäumraum in einem Inneren des Behälterkörpers definiert ist,
der Auschäumraum ein steriles Gas und eine Scheibe (30) enthält, die so konfiguriert ist, dass sie sich auf dem Boden des Behälterkörpers dreht, und die einen magnetischen Teil (33) und eine zum Mischen geeignete Form aufweist und so konfiguriert ist, dass sie funktionell mit einem Magnetrührer gekoppelt werden kann, ohne dass der Magnetrührer in den Auschäumraum eintritt, **dadurch gekennzeichnet, dass**
der Behälter ein weibliches Kupplungselement (15) mit einem Luer-Konus zum Zusammenstecken mit einer Spitze einer Spritze umfasst, wobei das weibliche Kupplungselement in einer der Seitenwände (14) des Behälterkörpers an oder nahe der Bodenfläche des Behälterkörpers angeordnet ist, so dass die Spritze zur Entnahme der geschäumten Sklerosierenden Zussamensetzung verwendet werden kann, und
einen Druckausgleicher (19, 20) zum Ausgleich eines Drucks innerhalb des Auschäumraums und außerhalb des Auschäumraums.

2. Behälter (10) nach Anspruch 1, wobei das weibliche Kupplungselement (15) einen Deckel (16) zum Verschließen der Kupplung aufweist.

3. Behälter nach einem der Ansprüche 1 bis 2, wobei das weibliche Kupplungselement (15) zum Zusammenstecken mit einer Spritze ferner ein Einwegventil zum Einführen einer flüssigen Sklerosierenden Zussamensetzung umfasst.

4. Behälter nach einem der Ansprüche 1 bis 3, wobei der Druckausgleicher (19, 20) eine Druckentlastung ist.

5. Behälter nach Anspruch 4, wobei die Druckentlastung eine Öffnung (19) im Behälterkörper und einen lösbaren Deckel (20) umfasst.

6. Behälter nach Anspruch 5, wobei sich die Öffnung (19) an der Oberseite (18) des Behälterkörpers befindet.

7. Behälter nach Anspruch 5 oder 6, wobei die Öffnung (19) einen Durchmesser von 1 mm - 3 mm hat.

8. Behälter (10) nach einem der Ansprüche 5 bis 7, wobei der abnehmbare Deckel (20) einen Griff (22) zum Abziehen des abnehmbaren Deckels aufweist.

9. Behälter (10) nach einem der Ansprüche 1 bis 8, wobei die Scheibe eine obere Scheibe und eine untere Scheibe umfasst, die so konfiguriert sind, dass sie miteinander gekoppelt werden können, wobei die obere und die untere Scheibe ein Gehäuse zur Aufnahme des magnetischen Teils definieren.

10. Kit zur Herstellung einer geschäumten Sklerosierenden Zussamensetzung, umfassend einen Behälter (10) nach einem der Ansprüche 1 bis 9, ferner umfassend eine Spritze, insbesondere eine mit einer flüssigen sklerosierenden Zussamensetzung vorgefüllte Spritze.

11. Verfahren zur Herstellung und Extraktion einer geschäumten sklerosierenden Zussamensetzung, umfassend:
Bereitstellung eines Behälters (10) mit:
einen versiegelten sterilen Behälterkörper (12) zum Aufschäumen, der eine oder mehrere Seitenwände (14) aufweist, die sich zwischen einer Oberseite (18) und einer Unterseite (11) des Behälterkörpers (12) erstrecken, wobei ein Aufschäumraum in einem Inneren des Behälterkörpers (12) definiert ist, wobei der Aufschäumraum ein steriles Gas und ein Mischelement (30) enthält, das so konfiguriert ist, dass es betriebsmäßig mit einem drehbaren Stellglied gekoppelt ist, ohne dass das Stellglied in den Aufschäumraum eintritt, wobei
der Behälter außerdem ein weibliches Kupplungselement (15) zum Zusammenfügen mit einer Spritze umfasst, **dadurch gekennzeichnet, dass** der Behälter einen Druckausgleicher (19, 20) zum Ausgleichen eines Drucks innerhalb des Auschäumraums und außerhalb des Auschäumraums umfasst;
und das Verfahren ferner umfassend:
Bereitstellung einer mit einer flüssigen sklerosierenden Zussamensetzung gefüllten Spritze;
Zusammenstecken (102) der Spritze mit dem weiblichen Kupplungselement (15) und Einspritzen (104) der flüssigen sklerosierenden Zussamensetzung in den Aufschäumraum;
Betätigung (106, 108, 110) des Stellglieds, um das Mischelement (30) zu drehen und eine geschäumte sklerosierenden Zussamensetzung zu erzeugen,
Extraktion (112) der erhaltenen geschäumten sklerosierenden Zussamensetzung.

12. Verfahren nach Anspruch 11, bei dem die Spritze mit dem weiblichen Kupplungselement (15) verbunden bleibt, während der Schaum erzeugt wird, und bei dem gegebenenfalls die erhaltene geschäumte Sklerosierenden Zussamensetzung durch Absaugen der Spritze extrahiert wird.

13. Verfahren nach Anspruch 11 oder 12, bei dem das Stellglied so betrieben wird (108), dass das Mischelement mit einer ersten Drehzahl von 5.000 U/min oder weniger gedreht wird, und vorzugsweise das Stellglied so gedreht wird, dass das Mischelement während einer ersten Zeitspanne mit einer ersten Drehzahl zwischen 2.000 und 4.500 U/min gedreht wird.

14. Verfahren nach Anspruch 13, wobei die erste Zeitspanne zwischen 15 und 90 Sekunden, insbesondere zwischen 30 und 75 Sekunden, insbesondere zwischen 30 und 60 Sekunden, beträgt.

15. Verfahren nach einem der Ansprüche 11 bis 14, wobei der Aktuator arbeitet (110), während die erhaltene geschäumte Sklerosierenden Zussamensetzung mit einer zweiten Geschwindigkeit extrahiert wird, wobei die zweite Rotationsgeschwindigkeit niedriger ist als die erste Rotationsgeschwindigkeit.

## Revendications

1. Récipient (10) pour la production d'une composition sclérosante en mousse, le récipient comprenant:
un corps de récipient stérile scellé (12) pour le moussage comprenant une ou plusieurs parois latérales (14) s'étendant entre un sommet (18) et un fond (11) du corps de récipient (12), un espace de moussage étant défini dans un intérieur du corps de récipient,
l'espace de moussage contenant un gaz stérile et un disque (30) configuré pour tourner sur le fond du corps du récipient et comprenant une partie magnétique (33) et une forme appropriée pour mélanger, et configuré pour être couplé de manière opérationnelle avec un agitateur magnétique sans que l'agitateur magnétique ne pénètre dans l'espace de moussage, **caractérisé en ce que**
le récipient comprend un élément d'accouplement femelle (15) ayant un cône Luer pour s'accoupler avec un embout d'une seringue, l'élément d'accouplement femelle étant situé dans l'une des parois latérales (14) du corps du récipient, au niveau ou près de la surface inférieure du corps du récipient de sorte que la seringue peut être utilisée pour extraire la composition sclérosante moussée et
un égaliseur de pression (19, 20) pour égaliser une pression à l'intérieur de l'espace de moussage et à l'extérieur de l'espace de moussage.

2. Récipient (10) selon la revendication 1, dans lequel l'élément de couplage femelle (15) comprend un couvercle (16) pour fermer le couplage.

3. Récipient selon l'une quelconque des revendications 1 - 2, dans lequel l'élément de couplage femelle (15) destiné à s'accoupler avec une seringue comprend en outre une valve à sens unique pour l'introduction d'une composition sclérosante liquide.

4. Récipient selon l'une quelconque des revendications 1 à 3, dans lequel l'égaliseur de pression (19, 20) est une libération de pression.

5. Récipient selon la revendication 4, dans lequel la libération de pression comprend un orifice (19) dans le corps du récipient et un couvercle amovible (20).

6. Récipient selon la revendication 5, dans lequel l'orifice (19) est situé au sommet (18) du corps du récipient.

7. Récipient selon la revendication 5 ou 6, dans lequel l'orifice (19) a un diamètre de 1 mm à 3 mm.

8. Récipient (10) selon l'une quelconque des revendications 5 à 7, dans lequel le couvercle amovible (20) comprend une poignée (22) pour retirer le couvercle amovible.

9. Récipient (10) selon l'une quelconque des revendications 1 - 8, dans lequel le disque comprend un disque supérieur et un disque inférieur, configurés pour être couplés l'un à l'autre, le disque supérieur et le disque inférieur définissant un compartiment pour loger la partie magnétique.

10. Kit pour la préparation d'une composition sclérosante en mousse comprenant un récipient (10) selon l'une quelconque des revendications 1 - 9, comprenant en outre une seringue, spécifiquement une seringue pré-remplie d'une composition sclérosante liquide.

11. Procédé de préparation et d'extraction d'une composition sclérosante expansée comprenant :
fournir un récipient (10) ayant :
un corps de récipient stérile scellé (12) pour le moussage comprenant une ou plusieurs parois latérales (14) s'étendant entre un sommet (18) et un fond (11) du corps de récipient (12), un espace de moussage étant défini dans un intérieur du corps de récipient (12), l'espace de moussage contenant un gaz stérile et un élément de mélange (30) configuré pour être couplé de manière opérationnelle avec un actionneur rotatif sans que l'actionneur ne pénètre dans l'espace de moussage, dans lequel
le récipient comprend en outre un élément de couplage femelle (15) pour s'accoupler avec une seringue, **caractérisé en ce que** le récipient comprend un égaliseur de pression (19, 20) pour égaliser une pression à l'intérieur de l'espace de moussage et à l'extérieur de l'espace de moussage ;
et le procédé comprend en outre :
fournir une seringue remplie d'une composition sclérosante liquide ;
accoupler (102) la seringue avec l'élément de couplage femelle (15) et injecter (104) la composition sclérosante liquide dans l'espace de moussage ;
actionner (106, 108, 110) l'actionneur pour faire tourner l'élément de mélange (30) et créer une composition sclérosante en mousse,
extraction (112) de la composition sclérosante expansée obtenue.

12. Procédé selon la revendication 11, dans lequel la seringue reste accouplée avec l'élément de couplage femelle (15) pendant que la mousse est créée, et éventuellement dans lequel la composition sclérosante moussée obtenue est extraite en aspirant la seringue.

13. Procédé selon la revendication 11 ou 12, dans lequel l'actionneur est actionné (108) pour faire tourner l'élément de mélange à une première vitesse de rotation de 5000 RPM ou moins, et de préférence faire tourner l'actionneur pour faire tourner l'élément de mélange à une première vitesse de rotation comprise entre 2000 et 4500 RPM pendant une première période de temps.

14. Procédé selon la revendication 13, dans lequel la première période de temps est comprise entre 15 et 90 secondes, spécifiquement entre 30 et 75 secondes, plus spécifiquement entre 30 et 60 secondes.

15. Procédé selon l'une quelconque des revendications 11 à 14, dans lequel l'actionneur fonctionne (110) pendant que la composition sclérosante moussée obtenue est extraite à une seconde vitesse, dans lequel la seconde vitesse de rotation est inférieure à la première vitesse de rotation.
